# EUROPEAN PATENT APPLICATION

(11) **EP 4 811 390 A1**
(43) Date of publication of application: **23.09.2026**
(21) Application number: 25164416.7
(22) Date of filing: 18.03.2025
(51) Int. Cl.: G16H 50/20, G16H 50/50

(54) **METHOD FOR PREDICTING A USER S GLYCEMIC STATE WITHIN A PREDEFINED PREDICTION TIME HORIZON AND METHOD FOR CONTROLLING AN INSULIN DELIVERY DEVICE USING A CONTROL UNIT COMPRISING A MODEL PREDICTIVE CONTROL (MPC) SYSTEM**

(71) Applicant: Universität Bern, 3012 Bern (CH)
(72) Inventor: Garcia-Tirado, José, 3012 Bern (CH); Naik, Vihangkumar, 3007 Bern (CH); Escorihuela-Altaba, Clara, 3018 Bern (CH); Manzoni, Eleonora, 3008 Bern (CH)
(74) Representative: Longchamp, Jean-Nicolas

(57) **Abstract**

The invention pertains to a computer- or processor-implemented method for predicting a user's glycemic state within a specified prediction time horizon. It involves estimating the user's current metabolic state using an Extended Kalman Filter, with hyperparameters derived from the user's historical continuous glucose monitoring data, insulin, and optionally meal records. The method further predicts the user's glycemic state utilizing a personalized glucose prediction model tailored for a specific demographic group. This model customization involves determining parameters and further refining n≥2 key parameters relevant to personalizing the model, utilizing the user's historical data and meal records for enhanced accuracy.

## Description

### Technical Field

The present invention relates to a method for predicting a user's glycemic state as well as controlling insulin delivery using computing technologies, specifically targeting the diabetic care field. The method incorporates a personalized glucose prediction model and an Extended Kalman Filter to estimate a user's metabolic and glycemic states using historical and current data from continuous glucose monitoring, insulin administration, and optionally meal records. It also describes a control unit , which is designed to optimize and regulate insulin delivery in response to predicted glycemic changes, ensuring the user's blood glucose levels remain within a target range.

### Background of the invention

The management of Type 1 Diabetes (T1D) is a complex and demanding task that requires continuous monitoring and precise control of blood glucose levels. This is crucial to prevent both acute complications, such as hypoglycemia and hyperglycemia, and long-term complications, including cardiovascular disease, neuropathy, and retinopathy. Traditionally, individuals with T1D have relied on manual methods of monitoring and insulin administration, which can be burdensome and prone to human error.

Recent advancements in technology have led to the development of Automated Insulin Delivery (AID) systems, which aim to alleviate some of these challenges by automating the process of insulin administration. These systems typically integrate continuous glucose monitors (CGMs) with insulin pumps, using algorithms to predict future glucose levels and adjust insulin delivery accordingly. However, the effectiveness of these systems is often limited by their reliance on population-based models or poorly individualized algorithms. Such models may not accurately reflect the unique physiological and lifestyle factors of individual users, leading to suboptimal insulin delivery decisions.

One of the primary limitations of current AID systems is their inability to fully capture the dynamic nature of glucose-insulin interactions. Most systems employ models, which may not adequately represent the complex physiological processes involved in glucose regulation. This can result in inaccurate predictions and control actions, compromising the system's ability to maintain glucose levels within a target range.

Furthermore, existing systems often lack the capability to adapt to changes in an individual's metabolic state over time. Factors such as stress, illness, physical activity, and dietary habits can significantly influence glucose metabolism, necessitating a more flexible and responsive approach to insulin delivery. Without the ability to personalize and continuously update the prediction and control models, these systems may fail to provide optimal glycemic control.

The goal of the present invention is therefore to propose novel methods and systems with which the above-mentioned drawbacks can be eliminated or at least greatly diminished.

### Summary of the invention

Thus, the object of the present invention is to provide methods and a unit for accurately predicting a user's glycemic state and determining optimal insulin dosage using a personalized model predicated on individual metabolic parameters.

According to the present invention, these objects are achieved in particular through the elements of the independent claims. Advantageous embodiments follow moreover from the dependent claims and the description.

In particular, the objects of the present invention are, in a first aspect, achieved by a computer- or processor-implemented method for predicting a user's glycemic state within a predefined prediction time horizon, the method comprising the following steps:
a. Estimating the user's current metabolic state based on a current glucose monitoring user's data by means of an Extended Kalman Filter, wherein the hyperparameters of the Extended Kalman Filter are inferred from user's historical continuous glucose monitoring data and insulin and, advantageously meal records, including meal timing and composition;
b. Predicting the user's glycemic state within the predefined prediction time horizon based on the estimated current metabolic state and a personalized glucose prediction model, wherein the parameters of the glucose prediction model are determined for a specific demographic group except for n parameters, where n≥2, identified as being most influential for user-specific model personalization, and wherein the said n parameters are inferred from user's historical continuous glucose monitoring data and insulin and, advantageously meal records, including meal timing and composition.

This allows for a more accurate and individualized prediction of glycemic states by integrating a wide range of user-specific data, including historical glucose monitoring, insulin usage, and advantageously meal records. The use of a personalized EKF provides a robust mechanism for estimating the current metabolic state by effectively integrating multiple data types, such as glucose levels and insulin administration. This integration results in a more precise representation of the user's metabolic state compared to traditional methods that rely on non-personalized models.

The personalization of the glucose prediction model further enhances the accuracy of glycemic state predictions. By identifying and tailoring the most influential parameters for each user, the model can account for individual physiological differences and lifestyle factors, leading to more precise predictions. This dual-step approach ensures that both the estimation of the metabolic state and the subsequent prediction of glycemic states are as accurate as possible, providing a significant improvement over generic models that may not adequately reflect individual variations.

In practical terms, this method can lead to better-informed decision-making for users with diabetes, such as optimizing dietary choices and insulin administration. By enhancing the accuracy and relevance of glycemic predictions, the method supports more effective management of blood sugar levels, potentially reducing the risk of complications associated with diabetes.

In a first preferred embodiment of the first aspect of the present invention, the most influential n parameters in the glucose prediction model are identified considering specific glucose-related states, such as postprandial and post-absorptive states, and events, such as used-induced correction boluses, using a time-dependent Sobol analysis.

This allows for a more precise and contextually relevant personalization of the glucose prediction model. By focusing on specific physiological states and user actions, the method can capture the dynamic nature of glucose metabolism more accurately. The time-dependent Sobol analysis evaluates the impact of each parameter over different times, which helps in understanding how glucose levels respond to various stimuli and conditions. This results in a model that is better tailored to the individual's distinct metabolic responses, leading to more accurate predictions of glycemic states. As a result, this enhances the effectiveness of insulin delivery systems by providing more reliable data for decision-making, improving blood glucose management for users with diabetes.

In another preferred embodiment of the first aspect of the present invention, the hyperparameters of the Extended Kalman Filter and/or the parameters of the glucose prediction model are continuously adapted based on user's continuous glucose monitoring, meal and insulin records.

This embodiment involves the continuous adaptation of the hyperparameters of the Extended Kalman Filter (EKF) and/or the parameters of the glucose prediction model based on the user's continuous glucose monitoring, meal, and insulin records. This dynamic adjustment allows the method to remain aligned with the user's current metabolic state, accounting for real-time changes in factors such as meal composition and insulin dosage. By continuously updating these parameters, the system can provide more accurate and responsive predictions of the user's glycemic state. This approach enhances the precision of the metabolic state estimation and glycemic predictions, leading eventually to more reliable insulin delivery decisions. The continuous adaptation ensures that the method can quickly respond to changes in the user's behavior or health status, such as variations in meal timing or insulin administration patterns, thereby maintaining optimal glycemic control. This method supports better-informed decision-making for users, improving the management of blood sugar levels and potentially reducing the risk of complications associated with diabetes.

In yet another preferred embodiment of the first aspect of the present invention, the glucose prediction model is the Hovorka glucose regulatory (GRM) model [1], the Subcutaneous Oral Glucose Minimal Model (SOGMM) [2], the Subcutaneous Oral Glucose Minimal Model (SOGMM) [3] or the Madgelaine's Model [4] .

The Hovorka model is a well-established framework that has been developed through extensive empirical data and clinical validation, providing a high degree of accuracy and biological plausibility in its predictions.

In a further preferred embodiment of the first aspect of the present invention, the hyperparameters of the Extended Kalman Filter, in particular the process noise covariance matrix Q, the measurement noise covariance matrix R, and/or the initial state covariance matrix *P₀* are inferred from user's historical continuous glucose monitoring data and insulin and meal records including meal timing and composition.

This approach allows the EKF to be finely tuned to the individual user's physiological and behavioral patterns, rather than relying on generic or fixed values. By tailoring these hyperparameters to the user's historical data, it is possible to more accurately model the user's metabolic state, leading to improved estimation accuracy. This personalization reduces the estimation errors that might arise from generic assumptions about noise levels or initial states, thereby enhancing the reliability of the metabolic state estimates.

Furthermore, the continuous adaptation of these matrices ensures that the EKF remains robust under dynamic and varying conditions, such as changes in meal timing, insulin administration patterns, and glucose variability. This adaptability makes the method more resilient to inconsistencies or noise in the input data, further enhancing the accuracy of metabolic state estimation. The synergy between the EKF and the glucose prediction model is strengthened, as both components are aligned with user-specific characteristics, improving the overall predictive accuracy. This alignment allows for more precise glycemic state predictions that account for individual variations in insulin sensitivity, meal effects, and other physiological factors, leading to more effective diabetes management.

In a second aspect, the objects of the present invention are achieved by a method for controlling an insulin delivery device using a control unit comprising a Model Predictive Control (MPC) system, wherein the method comprises the following steps:
a. Acquiring a user's current glucose level, preferably via a continuous glucose monitor;
b. Predicting the user's glycemic state within a specified prediction time horizon by means of the method of any one of the claims 1 to 5;
c. Determining optimal insulin dosage(s) within the specified prediction time horizon, to minimize glucose level deviations from a predefined target range;
d. Transmitting the determined optimal insulin dosage(s) to the insulin delivery device for administration to the user;
wherein steps a. to d. are repeated at a predetermined frequency.

The integration of real-time glucose monitoring with advanced glycemic state prediction enables a dynamic and responsive insulin delivery system. By continuously acquiring glucose levels and predicting future glycemic states by means of the method of the present invention, which provides a user-specific prediction model, the system can adjust for user-specific insulin dosage in real-time, ensuring that blood glucose levels remain within a predefined target range. This adaptability is important for managing the user-specific fluctuations in glucose levels that occur due to various factors such as meals, physical activity, and stress.

The use of a Model Predictive Control system enables the calculation of optimal insulin dosages by considering both current glucose levels and predicted trends. This approach minimizes deviations from target glucose ranges, enhancing user comfort and reducing the risk of complications associated with diabetes, such as hypoglycemia and hyperglycemia. The method's iterative nature ensures that insulin delivery is continuously optimized, providing a more stable and effective user-specific management of blood sugar levels.

In a first preferred embodiment of the second aspect of the present invention, the glucose prediction model is a nonlinear model and wherein for each prediction of the user's glycemic state at step b., the model is linearized around the estimated current metabolic state [5].

This allows for a more accurate representation of the complex biological systems involved in glucose regulation, such as insulin dynamics and meal effects, which are often characterized by nonlinear relationships. By linearizing the model around the estimated current metabolic state, the system can make precise predictions within the specified prediction time horizon, minimizing errors that may arise from assuming linear behavior in biological systems while keeping the computing resources needed low. This iterative process of re-linearizing the model as the user's glycemic state evolves ensures that predictions remain accurate and relevant, enhancing the overall performance of the method. The integration of this linearized model with the MPC system allows for real-time optimization of insulin delivery, taking into account both current glucose levels and predicted trends, resulting in more effective glycemic control. This approach anticipates changes in blood sugar levels and responds accordingly, providing a dynamic and responsive system that adapts to the user's physiological state.

In another preferred embodiment of the second aspect of the present invention, the prediction of the user's glycemic state at step b. incorporates user-provided meal announcements data.

This allows the method to refine predictions by considering real-time user dietary inputs, which are important for accurately forecasting blood glucose fluctuations. By utilizing meal timing and composition data directly from the user, the method can adjust insulin delivery strategies to better anticipate and respond to the glycemic impact of upcoming meals. This approach enhances the precision of glycemic predictions by aligning them with the user's actual dietary habits, rather than relying solely on historical data or generic assumptions. As a result, the method can make more informed adjustments to insulin dosages, particularly around mealtimes, thereby minimizing deviations from target glucose levels. This user-centric method improves the adaptability and responsiveness of the insulin delivery system, leading to more effective blood glucose management and potentially reducing the risk of postprandial hyperglycemia. Additionally, the seamless integration of meal announcements simplifies user interaction, making the method more intuitive and practical for daily use.

In yet another preferred embodiment of the second aspect of the present invention, the optimal insulin dosage(s) determined at step c. is computed taking into account eventual user's manual insulin injection(s) and/or insulin injection(s) triggered by a hyperglycemia supervisor module of the control unit.

This correction mechanism ensures that the insulin delivery system remains responsive to both automated and user-initiated inputs, thereby enhancing the precision of insulin administration. By integrating manual and automated insulin delivery actions, the method can maintain a more accurate balance of insulin levels, reducing the risk of hypoglycemia or hyperglycemia. This approach allows for a more comprehensive management of blood glucose levels by considering all sources of insulin input, leading to improved glycemic control. The correction step ensures that the method adapts to real-time changes in insulin needs, providing a more reliable and user-friendly experience for individuals managing diabetes.

In a further preferred embodiment of the second aspect of the present invention, the method further comprises a step of evaluating the determined optimal insulin dosage(s) against one or more safety thresholds prior to transmission to the insulin delivery device for administration.

This step introduces a safety mechanism into the insulin delivery process, ensuring that all calculated dosages are within safe and acceptable limits. By incorporating this evaluation, the method can prevent potential errors such as overdosing or underdosing, which are significant in diabetes management where precise dosing is necessary to avoid complications like hypoglycemia or hyperglycemia. This additional layer of verification enhances the reliability and trustworthiness of the method, providing assurance that all dosages meet safety criteria before administration. The integration of safety thresholds ensures that the method remains robust and reliable, adapting to individual user needs while maintaining optimal glycemic control.

In another preferred embodiment of the second aspect of the present invention, the method further comprises:
i. Maintaining an estimate of Insulin On Board representing the residual effect of recently administered insulin;
ii. At step c., incorporating the estimate of Insulin On Board, the current glucose level and the current glucose rate of change as constraint, to avoid excessive insulin stacking; and
iii. Updating the estimate of Insulin On Board after each administration at step d.

An IOB estimator provides a semi-continuous estimate of the amount of active insulin remaining in the body from previous insulin injections. In the computation, it compensates for the diffusion lag between plasma glucose concentration and glucose concentration in interstitial tissues, and takes into account the current glucose value and rate of change (trend) to estimate the insulin action exponential decay curve. Finally, a penalty factor tracks the changes in insulin sensitivity related to the circadian rhythm.

By maintaining an estimate of IOB, the method can accurately account for the residual effects of previously administered insulin, which is important for preventing insulin stacking-a condition where overlapping insulin doses lead to excessive insulin levels in the body. This approach ensures that the method can make more informed decisions about future insulin dosages, reducing the risk of hypoglycemia caused by excessive insulin administration.

Incorporating the IOB estimate, along with the current glucose level and rate of change, as constraints in the dosage determination process allows the method to dynamically adjust insulin delivery in response to real-time physiological data. This integration enhances the precision of insulin dosing by considering the ongoing metabolic state of the user, leading to more stable blood glucose control.

Updating the IOB estimate after each insulin administration ensures that the method continuously adapts to the user's current insulin needs, maintaining an accurate representation of insulin activity over time. This real-time adaptability is important for managing the dynamic nature of glucose metabolism, particularly in response to factors such as meals, physical activity, and stress.

Finally, by incorporating the estimate of Insulin On Board, the current glucose level and the current glucose rate of change, it is possible to discourage sudden or large changes in insulin dosage by introducing and continuously adapting a penalty on rate of change of optimal insulin dosage in the determination process at step c.

In a third aspect, the invention relates to a control unit communicatively coupled to an insulin delivery system and a continuous glucose monitoring device and comprising means adapted to execute the steps of the method of the second aspect of the present invention.

This also allows the integration of real-time glucose monitoring with advanced glycemic state prediction enabling a dynamic and responsive insulin delivery system. With a control unit communicatively coupled to an insulin delivery system, such as an insulin pump, and a continuous glucose monitoring device and comprising means adapted to execute the steps of the method of the second aspect of the present invention, it is possible to continuously acquire glucose levels and predict future glycemic states. The control unit can thus adjust for user-specific insulin dosage in real-time, ensuring that blood glucose levels remain within a predefined target range. This adaptability is important for managing the user-specific fluctuations in glucose levels that occur due to various factors such as meals, physical activity, and stress.

The use of a Model Predictive Control system enables the calculation of optimal insulin dosages by considering both current glucose levels and predicted trends. This approach minimizes deviations from target glucose ranges, enhancing user comfort and reducing the risk of complications associated with diabetes, such as hypoglycemia and hyperglycemia.

The integration with existing systems is another notable benefit, as it allows for seamless interaction between the control unit, the insulin pump, and the glucose monitor. This connectivity enables the system to utilize the latest data for predictions and decisions, ensuring optimal performance and accuracy over time. Additionally, the use of nonlinear models in combination with personalized EKF, as detailed above, allows to improve the precision of glycemic predictions. This reduction in prediction errors can help minimize the risks associated with hypo- and hyper-glycemic episodes, making the control unit more reliable for users with diabetes. The ability to adapt dosages not just according to static thresholds but also to personal health data can lead to a more customized and effective treatment plan.

In further aspects, the present invention relates to a computer program product comprising instructions to cause the control unit of the present invention to execute the steps of the method according to the second aspect of the present invention and to a computer-readable medium having stored thereon the computer program of the present invention.

### Brief description of the drawings

- Figure 1 illustrates a flowchart depicting the workflow of a computer- or processor implemented method for predicting a user's glycemic state within a predefined prediction time horizon according to a preferred embodiment of the first aspect of the present invention;
- Figure 2 illustrates a flowchart outlining the method for controlling an insulin delivery device using a control unit comprising a Model Predictive Control (MPC) system according to a preferred embodiment of the present invention;
- Figure 3 presents a schematic diagram of a control unit according to a preferred embodiment of the present invention;
- Figure 4 illustrates the Hovorka glucose-insulin model;
- Figure 5 shows sensitivity analysis of Hovorka model for one participant from an in-silico dataset over two days;
- Figure 6 shows sensitivity analysis of Hovorka model for one participant from a real dataset over two days;
- Figure 7 shows the ranking of parameters of Hovorka model using in-silico data averaged over two days; and
- Figure 8 shows the ranking of parameters of Hovorka model using real data averaged over two days.

### Detailed description of preferred embodiments and presentation of experimental results

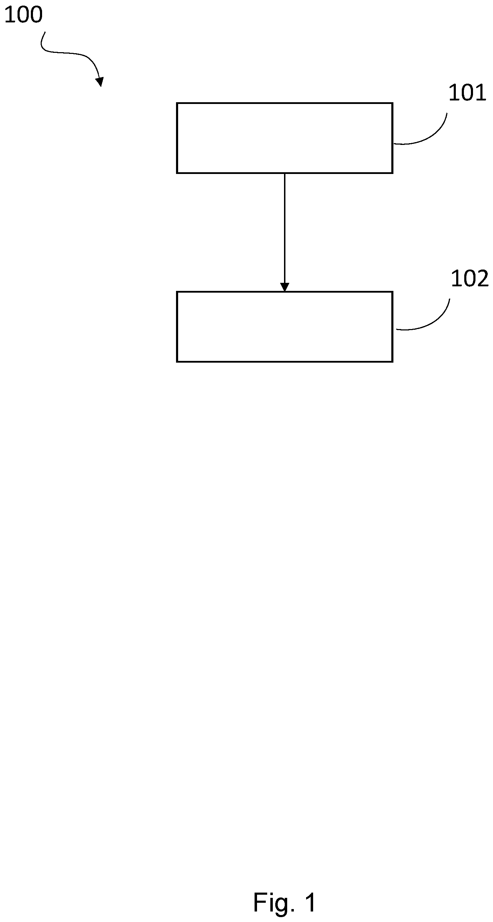

Figure 1 illustrates a flowchart depicting the workflow of a computer- or processor implemented method 100 for predicting a user's glycemic state within a predefined prediction time horizon according to a preferred embodiment of the first aspect of the present invention.

The workflow begins at step 101 with estimating the user's current metabolic state based on a current glucose monitoring user's data by means of an Extended Kalman Filter. The hyperparameters of the Extended Kalman Filter are inferred from user's historical continuous glucose monitoring data and insulin and, advantageously meal records, including meal timing and composition. The glucose monitoring user's data is obtained continuously from a Continuous Glucose Monitoring (CGM) device.

Subsequently, at step 102, the user's glycemic state within the predefined prediction time horizon is predicted based on the estimated current metabolic state and a personalized glucose prediction model. The parameters of the glucose prediction model are determined for a specific demographic group except for n parameters, where n≥2, identified as being most influential for user-specific model personalization. The said n parameters are inferred from user's historical continuous glucose monitoring data and insulin and, advantageously meal records, including meal timing and composition. The glucose prediction model is advantageously the Hovorka glucose regulatory model [1].

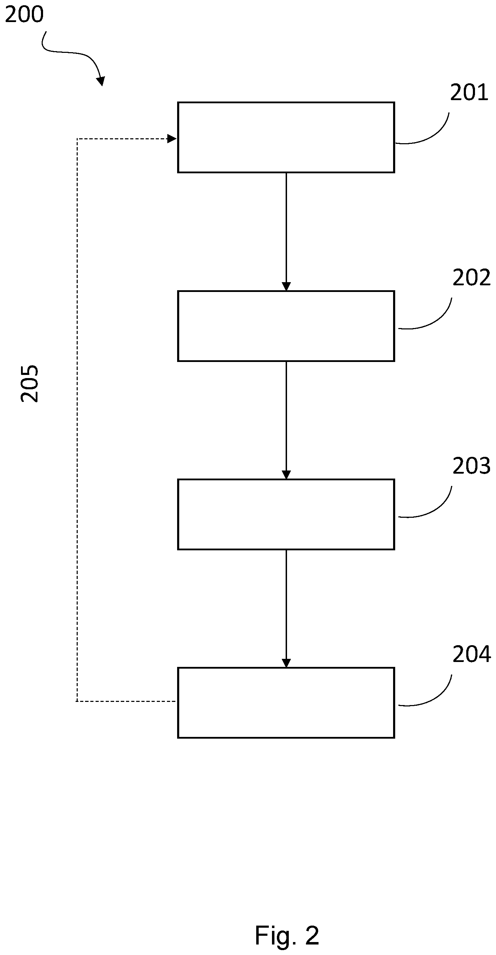

Figure 2 illustrates a flowchart outlining the method 200 for controlling an insulin delivery device using a control unit comprising a Model Predictive Control (MPC) system according to a preferred embodiment of the present invention. At step 201 user's current glucose level is acquired via a continuous glucose monitor. At step 202, the user's glycemic state within a specified prediction time horizon is predicted by means of the method of Figure 1. At step 203, optimal insulin dosage(s) within the specified prediction time horizon is determined, to minimize glucose level deviations from a predefined target range. Finally, at step 204, the determined optimal insulin dosage(s) is transmitted to the insulin delivery device for administration to the user. As illustrated by the dashed arrow 205, steps 201 to 204 are repeated at a predetermined frequency.

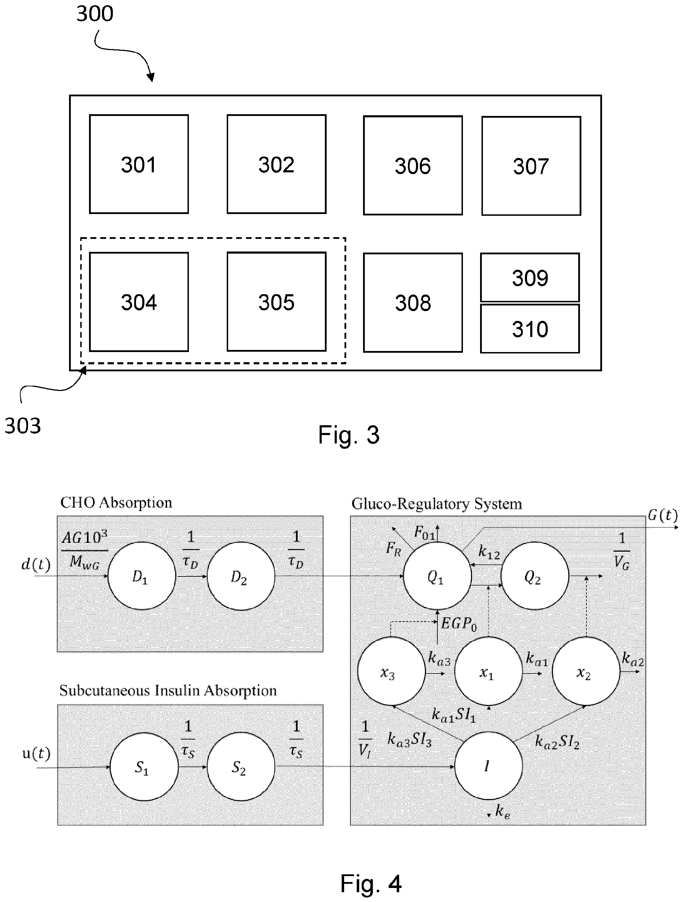

Figure 3 presents a schematic diagram of a control unit comprising a Model Predictive Control (MPC) system 300 communicatively coupled to an insulin delivery system and a continuous glucose monitoring device according to a preferred embodiment of the present invention. The control unit 300 comprises a processor 301, responsible for executing instructions stored in memory 302 to perform the steps of the described methods. The memory 302 serves as a storage unit, housing executable instructions for method execution, intermediate data results, and other relevant information. It can consist of various types of volatile and non-volatile memory technologies, such as random-access memory (RAM), flash memory, or hard disk drives. The unit features a user-friendly user interface 303 that facilitates interaction between users and the unit 300. The user interface comprises a display 304, typically a monitor or touchscreen device, for visualizing for instance current glucose levels and scheduled insulin deliveries. Input devices 305, such as keyboard, or touchpad, enabling users to manually enter information on manual insulin bolus or meal intakes are provided.

To facilitate data exchange between the unit 300 and a CGM and an insulin delivery device a communication module 306 is integrated. This interface enables the receipt and the transmission to these external devices.

Moreover, the control unit 300 includes a hyperglycaemia supervisor module 307, which can trigger immediate insulin injection if the glucose level measure by the CGM is above a predetermined threshold. The unit 300 comprises further an IOB estimator module 308 as well as a check point module 309 for evaluating the determined optimal insulin dosage(s) against one or more safety thresholds prior to transmission to the insulin delivery device for administration. Finally, the unit 300 comprises a bolus module 310 for manual insulin injection.

The unit 300 can be implemented using any suitable hardware and software technologies and can function either as an independent standalone system or integrated into larger systems, depending on specific use cases and deployment requirements.

A more detailed description of the personalization process for the glucose prediction model, using the Hovorka model as an example, in particular the determination of the most influential parameter of the model for its personalisation, is provided below.

The Hovorka glucose-insulin model, a control-oriented compartmental mathematical model for glucose prediction, is illustrated in Figure 4. It consists of three sub-models: (i) the subcutaneous insulin absorption sub-model, which describes insulin transport within the subcutaneous system, (ii) the glucose-regulatory system sub-model, which characterizes the physiological regulation of glucose in the body [1], and (iii) the carbohydrate (CHO) absorption sub-model proposed in [6].

The model can be defined as: ${\dot{x}}_{c} \left(t\right) = f \left({x}_{c}\left(t\right),{θ}_{H},{u}_{c}\left(t\right)\right)$ ${\hat{y}}_{c} \left(t\right) = g \left({x}_{c}\left(t\right),{θ}_{H}\right)$ where *x_{c}*(*t*) are the states, *θ_{H}* the parameters, *ŷ_{c}*(*t*) the outputs, and *u_{c}*(*t*) the inputs. The mathematical equations are described from (1) to (Eq. (16)), and Table 1 details the inputs, states, and parameters of the model. $\frac{{dQ}_{1} \left(t\right)}{dt} = {U}_{G} \left(t\right) - {x}_{1} \left(t\right) {Q}_{1} \left(t\right) - {F}_{01}^{c} \left(t\right) - {F}_{R} \left(t\right) + {k}_{12} {Q}_{2} \left(t\right) + {EGP}_{0} \left[1-{x}_{3}\left(t\right)\right]$ $\frac{{dQ}_{2} \left(t\right)}{dt} = {x}_{1} \left(t\right) {Q}_{1} \left(t\right) - \left[{k}_{12}+{x}_{2}\left(t\right)\right] {Q}_{2} \left(t\right)$ $\frac{{dx}_{1} \left(t\right)}{dt} = - {k}_{{a}_{1}} {x}_{1} \left(t\right) + {k}_{{b}_{1}} I \left(t\right)$ $\frac{{dx}_{2} \left(t\right)}{dt} = - {k}_{{a}_{2}} {x}_{2} \left(t\right) + {k}_{{b}_{2}} I \left(t\right)$ $\frac{{dx}_{3} \left(t\right)}{dt} = - {k}_{{a}_{3}} {x}_{3} \left(t\right) + {k}_{{b}_{3}} I \left(t\right)$ $\frac{{dS}_{1} \left(t\right)}{dt} = u \left(t\right) - \frac{{S}_{1} \left(t\right)}{{τ}_{S}}$ $\frac{{dS}_{2} \left(t\right)}{dt} = \frac{{S}_{1} \left(t\right)}{{τ}_{S}} - \frac{{S}_{2} \left(t\right)}{{τ}_{S}}$ $\frac{dI \left(t\right)}{dt} = \frac{{S}_{2} \left(t\right)}{{τ}_{S} {V}_{I}} - {k}_{e} I \left(t\right)$ $\frac{{dD}_{1} \left(t\right)}{dt} = \frac{1000 AG}{{M}_{wg}} d \left(t\right) - \frac{{D}_{1} \left(t\right)}{{τ}_{D}}$ $\frac{{dD}_{2}}{dt} = \frac{{D}_{1} \left(t\right)}{{τ}_{D}} - \frac{{D}_{2} \left(t\right)}{{τ}_{D}}$ $G \left(t\right) = \frac{{Q}_{1} \left(t\right)}{{V}_{G}}$ ${F}_{01}^{c} \left(t\right) = \left\{\begin{matrix}{F}_{01} , & G \left(t\right) \geq 4.5 mmol / L \\ \frac{{F}_{01} G \left(t\right)}{4.5} , & G \left(t\right) < 4.5 mmol / L\end{matrix}\right.$ ${F}_{R} \left(t\right) = \left\{\begin{matrix}0.003 \left(G\left(t\right)-9\right) {V}_{G} , & G \left(t\right) \geq 9 mmol / L \\ 0 , & G \left(t\right) < 9 mmol / L\end{matrix}\right.$ Where, ${SI}_{i} = \frac{{k}_{{a}_{i}}}{{k}_{{b}_{i}}} ∀ i = 1 , 2 , 3 .$

**Table 1. Inputs, outputs, states and parameters details for Hovorka glucose prediction model**

| Name | Description | Unit |
|---|---|---|
| Inputs (*u_{c}*(*t*)) | | |
| u(t) | Units of insulin per minutes | mU/min |
| d(t) | Grams of CHO per minute | g/min |

| Output (*ŷ*(*t*)) | | |
|---|---|---|
| *G* (*t*) | Glucose concentration in blood stream | *mmol*/*L* |

| States (*x_{c}*(*t*)) | | |
|---|---|---|
| *Q*₁ (*t*) | Glucose mass in the accessible compartment | *mmol* or *mmol*/*kg* |
| *Q*₂ (*t*) | Glucose mass in the non-accessible compartment | *mmol* or *mmol*/*kg* |
| *x*₁ (*t*) | Effects of insulin on glucose distribution/transport | *min*⁻¹ |
| *x₂* (*t*) | Effect of insulin on glucose disposal | *min*⁻¹ |
| *x*₃ (*t*) | Effect of insulin on Endogenous glucose production | *min*⁻¹ |
| *S*₁(*t*) | Short-acting insulin subsystem | *mU* or *mU*/*kg* |
| *S*₂ (*t*) | Short-acting insulin subsystem | *mU* or *mU*/*kg* |
| *I* (*t*) | Plasma insulin concentration | *mU*/*L* |
| *D*₁ (*t*) | CHO absorption compartment 1 | *mmol* or *mmol*/*kg* |
| *D*₂ (*t*) | CHO absorption compartment 2 | *mmol* or *mmol*/*kg* |

| Parameters (*θ_{H}*) with population values | | |
|---|---|---|
| *k*₁₂ | Transfer rate | *min*⁻¹ |
| *EGP*₀ | Endogenous glucose production extrapolated to zero insulin concentration | *mmol kg⁻¹ min*⁻¹ |
| *k*_{*a*₁} | Deactivation rate | *min*⁻¹ |
| ${SI}_{1} = \frac{{k}_{{b}_{1}}}{{k}_{{a}_{1}}}$ | Insulin sensitivity of distribution/transport | *min*⁻¹ *per L*/*mU* |
| *k*_{*a*₂} | Deactivation rate | *min*⁻¹ |
| ${SI}_{2} = \frac{{k}_{{b}_{2}}}{{k}_{{a}_{2}}}$ | Insulin sensitivity of disposal | *min*⁻¹ *per L*/*mU* |
| *k*_{*a*₃} | Deactivation rate | *min*⁻¹ |
| ${SI}_{3} = \frac{{k}_{{b}_{3}}}{{k}_{{a}_{3}}}$ | Insulin sensitivity of EGP | *min*⁻¹ *per L*/*mU* |
| *τ_{S}* | Insulin absorption constant | *min* |
| *kₑ* | Insulin elimination rate | *min*⁻¹ |
| *AG* | Carbohydrate availability | *NA* |
| *M_{wg}* | Glucose molecular weight | *g*/*mol* |
| *τ_{D}* | CHO absorption rate | *min* |
| *V_{G}* | Glucose distribution volume | *L*/*kg* |
| *V_{I}* | Insulin distribution volume | *L*/*kg* |
| *F*₀₁ | Non-insulin dependent glucose flux | *mmol kg*⁻¹ *min*⁻¹ |

Phenomenological models stand out due to their descriptive capacity. However, while population-level parameters are documented, identifying which parameters impact the model's output the most is essential for personalization. This personalization process improves prediction accuracy. Below a personalization methodology based on the Sobol sensitivity analysis is presented.

The Sobol method is a well-established global sensitivity analysis technique that quantifies the contribution of input variables to the variance of a model's output. This method decomposes the total variance into fractions and attributes them to the model's inputs (Eq. (17)). A model is considered more sensitive to those inputs whose contribution results in a higher impact on the total variance [7]. $V \left(Y\right) = {\sum }_{i = 1}^{n} {V}_{{θ}_{i}} + {\sum }_{i < j}^{n} {V}_{{θ}_{i} , {θ}_{j}} + ⋯ + {V}_{{θ}_{1} , {θ}_{2} , … , {θ}_{n}}$

Here, the impact of the model parameters (Eq. (18)) and their possible interactions (Eq. (19)) in the total variance of the output are considered. Based on this, Sobol indices are computed as presented in [8]. Two Sobol indices are used: first-order Sobol indices (*S_{θᵢ}*), which measure the contribution of single parameters (*θᵢ*) to the variance of the model by (Eq. (20)), and total-order Sobol indices ( ${S}_{{T}_{{θ}_{i}}}$), calculated as (Eq. (21), which account for both single parameters (*θᵢ*) and all their interactions. ${V}_{{θ}_{i}} = {V}_{{θ}_{i}} \left({E}_{{θ}_{∼ i}}\left[\left.Y\right|{θ}_{i}\right]\right) ,$ ${V}_{{θ}_{i} , {θ}_{j}} = {V}_{{θ}_{i}} \left({E}_{{θ}_{∼ i}}\left[\left.Y\right|{θ}_{i},{θ}_{j}\right]\right) - {V}_{{θ}_{i}} - {V}_{{θ}_{j}} ,$ ${S}_{{θ}_{i}} = \frac{{V}_{{θ}_{i}}}{V \left(Y\right)} , {S}_{{θ}_{i}} ∈ \left[0, 1\right] ∀ i ∈ \left\{1,…,n\right\}$ ${S}_{{T}_{{θ}_{i}}} = {S}_{{θ}_{i}} + {\sum }_{\begin{matrix}j ϵ \left\{1,…,n\right\} \\ j \neq i\end{matrix}} {S}_{{θ}_{i} , {θ}_{j}} + ⋯ + {S}_{{θ}_{1} , {θ}_{2} , … , {θ}_{n}} = 1 - \frac{{Var}_{{θ}_{∼ i}} \left({E}_{{θ}_{i}}\left[\left.Y\right|{θ}_{∼ i}\right]\right)}{Var \left(Y\right)}$

In practical cases, these indices are typically estimated using a Monte Carlo approach. Based on this, the specific methodology used here is detailed in [9].

Sobol indices are typically computed for models that do not account for time dependency. To adapt them for a time-dependent model, first-order (*S_{tₛ,θᵢ}*) and total-order ( ${S}_{{T}_{{t}_{s} , {θ}_{i}}}$) Sobol indices at each time step (*tₛ*) of the prediction are computed.

Since model variance changes over time, typically increasing as the prediction horizon progresses, a direct comparison of the indices is not straightforward. To address this, each Sobol index is normalized as in (Eq. (22)) to (Eq. (24)), ${S}_{{t}_{s} , {θ}_{i}}^{∗} = {w}_{{t}_{s}} {S}_{{t}_{s} , {θ}_{i}} , {S}_{{t}_{s} , {θ}_{i}}^{∗} ∈ \left[0, 1\right] , ∀ i ∈ \left\{1,…,n\right\}$ ${S}_{{T}_{{t}_{s} {θ}_{i}}}^{∗} = {w}_{{t}_{s}} {S}_{{T}_{{t}_{s} , {θ}_{i}}} , {S}_{{T}_{{t}_{s} {θ}_{i}}}^{∗} ∈ \left[0, 1\right] , ∀ i ∈ \left\{1,…,n\right\}$ **With** *w_{tₛ}* defined as, ${w}_{{t}_{s}} = \frac{{V}_{{t}_{s}} \left(Y\right)}{{max}_{{t}_{s} \leq PH} {V}_{{t}_{s}} \left(Y\right)} , {w}_{{t}_{s}} ∈ \left[0, 1\right] ,$ and then average as in (Eq. (25)) and (Eq. (26)) to obtain a single first (*S̅_{θᵢ}*) and total ( ${\overline{S}}_{{T}_{{θ}_{i}}}$) order Sobol index value for the entire prediction: ${\overline{S}}_{{θ}_{i}} = \frac{1}{PH} {\sum }_{{t}_{s} = 1}^{PH} {S}_{{t}_{s} , {θ}_{i}}^{∗} , {\overline{S}}_{{θ}_{i}} ∈ \left[0, 1\right] , ∀ i ∈ \left\{1,…,n\right\}$ ${\overline{S}}_{{T}_{{θ}_{i}}} = \frac{1}{PH} {\sum }_{{t}_{s} = 1}^{PH} {S}_{{T}_{{t}_{s} {θ}_{i}}}^{∗} , {\overline{S}}_{{T}_{{θ}_{i}}} ∈ \left[0, 1\right] , ∀ i ∈ \left\{1,…,n\right\} .$

Finally, parameters are ranked based on their *S̅_{θᵢ},* with higher values indicating a more significant influence on the model variance.

This approach was applied to identify the most influential parameters in the Hovorka GRM. Numerical analyses were performed using simulated (in-silico) and real data from three adults with T1D each. Data was collected over 10 days, including CGM measurements, insulin records, and meal intake.

In-silico data is generated from a modified version of the UVA/Padova simulator [10] incorporating insulin sensitivity variability and random meal in- take on participants with an open-loop insulin-infusion system. Real data comes from a randomized crossover clinical trial, where participants in free-living conditions used a closed-loop AID system. 576 data points from in-silico and real dataset were used.

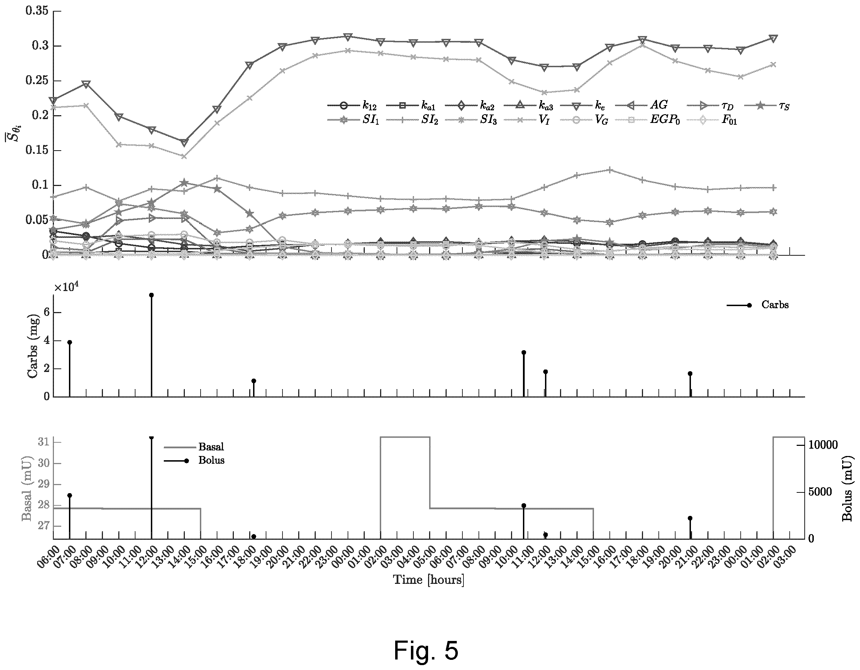

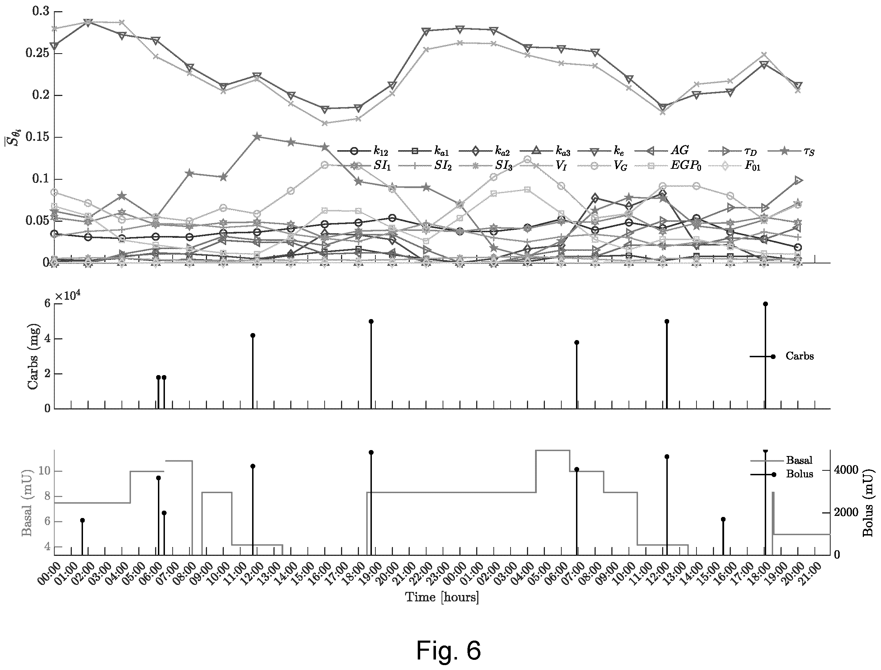

Figures 5 and 6 show the results of the proposed approach on in-silico and real data, respectively. The top panel in both figures show the estimated values of*S̅_{θᵢ}*. Values for ${\overline{S}}_{{T}_{{θ}_{i}}}$ are not shown for simplicity, however their values are always slightly higher and the dynamic trend is the same as for *S̅_{θᵢ}*. In both figures, the middle panel illustrates the CHO content of the meals, and the bottom panel shows insulin infusions, with vertical gray lines indicating the 2-hour time windows where predictions were made.

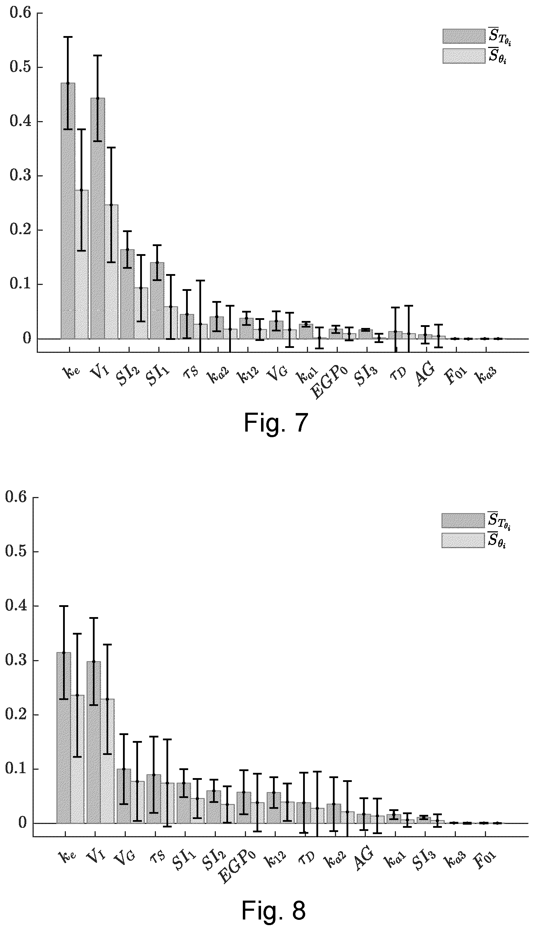

Even though Figures 5 and 6 provide a clear illustration of the model's sensitivity to parameters over the studied time-lapse, it is not straightforward to obtain a global ranking of the parameters visually. To address this, Figures 7 and 8 show the average parameter impact on the model's output, for in-silico and real datasets, considering the entire data by the *S̅_{θᵢ}^{avg}* and the ${{\overline{S}}_{{T}_{{θ}_{i}}}}^{avg}$ metrics. Note that the y-axis of top panels is unitless, as the indices are dimensionless.

These results show that ${\overline{S}}_{{T}_{{θ}_{i}}}$ is consistently slightly higher than *S̅_{θᵢ}* index across all parameters in both simulated and real data. This is expected as ${\overline{S}}_{{T}_{{θ}_{i}}}$ should be higher than *S̅_{θᵢ}* ; however, the slight difference between both indices suggests that the variance of the model is mainly caused by the individual contribution of the parameters, rather than by their interactions. The volume of insulin distribution (*V_{I}*) and insulin elimination from plasma (*kₑ*) are the two key parameters with higher *S̅_{θᵢ}* and ${\overline{S}}_{{T}_{{θ}_{i}}}$ indices in both datasets. Nevertheless, their importance decreases as insulin sensitivity of distribution/transport (SI₁), insulin sensitivity of disposal (SI₂), CHO absorption constant (*τ_{D}*), insulin absorption constant (*τ_{S}*), and glucose distribution volume (*V_{G}*) become more relevant at specific times, especially during postprandial events, indicating that parameter relevance depends on meal intake. This sensitivity analysis provides a systematic means of highlighting/raking significance of parameters for the given participant data during different metabolic conditions. Based on the underlying results, a choice can be made to determine the number of parameters to be personalized. In this case, six parameters are identified as the main parameters for model personalization. The three most influential parameters during post-absorptive periods (*kₑ, V_{I}* , and SI₂) to fit baseline glucose levels, and the three most influential in postprandial stages (*τ_{D}, τ_{S},* and *V_{G}*) to fit the onset and end of this phase. It is important to understand that the relevance of parameters computed by the time-dependent Sobol method is highly dependent on the richness of the data. For instance, a dataset with a high number of meal events will highlight the significance of parameters related to the meal compartment more than a dataset with a lower number of meal events. Thus, given that the parameter ranking is different depending on the richness of the data, it is important to determine the subset of influential parameters to calibrate in each dataset. Selecting the optimal parameter subset to calibrate ensures proper personalization and improves accuracy in glucose predictions.

To assess the impact of their personalization, the performance of the model was analysed by using three different sets of parameters: (a) Population, where all parameters used population values as per Hovorka et al. [1]; Wilinska et al. [3]; (b) Arbitrary, where parameters used population values except for a set of parameters (*θ_{A} =* (*τ_{D}*, *F*₀₁*, k*_{*a*₃}*, AG, EGP*₀, *k*₁₂)) that were arbitrarily selected for personalization, and (c) SA, where parameters used population values except for the parameters chosen through our proposed sensitivity analysis (*θ_{SA}* = (*kₑ, V_{I}* , SI₂, *τ_{D}, τ_{S}, V_{G}*)).

Personalization was performed using data from seven days with a nonlinear least-squares solver in MATLAB, while the remaining data from three days were used for validation. 2-h glucose predictions were computed using the Hovorka GRM. Table 2 presents the median[IQR] for the Root Mean Squared Error (RMSE) (mg/dL) over the entire prediction in the validation data for three participants in both the simulated and real datasets.

**Table 2**

| | **Calibration Strategy** | | |
|---|---|---|---|
| **Data** | **Population** | **Arbitrary** | **SA** |
| **Simulated** | 40.2 [22.9, 62.0] | 7.7 [3.9, 20.4] | 4.6 [2.1, 9.9] |
| **Real** | 34.2 [18.4, 59.6] | 26.8 [15.5, 41.6] | 23.5 [14.4, 40.0] |

Compared to population values, an arbitrary set of calibrated parameters reduces RMSE by 80% and 22% of RMSE for simulated and real data, respectively. However, personalizing parameters based on the proposed sensitivity analysis achieved reductions of 90% and 32% for simulated and real data respectively, demonstrating that selecting the most influential parameters significantly enhances the prediction capability of the model under consideration.

Finally, the decrease in the RMSE between the calibration of *θ_{A}* (arbitrary) and *θ_{SA}* (sensitivity analysis) parameters is similar for simulated and real data (≈ 10%). However, the overall improvement in the RMSE when comparing the personalization of *θ_{H}* (population) and the *θ_{SA}* parameters is lower for the real data. This is due to other sources of variability unrelated to the physical systems that the calibrated parameters are intended to explain

Adapting Sobol indices to a dynamic time series shows that parameters that primarily influence the output's variability of physiological models change under varying dynamic conditions. By calibrating the parameters ranked by the time-dependent Sobol index, the prediction accuracy rendered a reduction in RMSE by 90% and 32% in simulated and real data respectively. This demonstrates the advantage to apply the time-dependent Sobol index for parameter identification, improving glucose prediction accuracy in any physiological models and enhancing model-based digital therapeutics performance.

### References

[1] R. Hovorka et al., "Nonlinear model predictive control of glucose concentration in subjects with type 1 diabetes," Physiol Meas, vol. 25, no. 4, pp. 905-920, Aug. 2004, doi: 10.1088/0967-3334/25/4/010.
[2] Patek SD, Lv D, Ortiz EA, et al. Empirical representation of blood glucose variability in a compartmental model. In: Kirchsteiger H, Jorgenson JB, Renard E, del Re L, eds. Prediction Methods for Blood Glucose Concentration. Cham: Springer; 2016:133-157.
[3] Corbett, J.P., Colmegna, P., Garcia-Tirado, J. and Breton, M.D., 2020. Anticipating meals with behavioral Profiles in an artificial pancreas system-An informed multistage model predictive control approach. IFAC-PapersOnLine, 53(2), pp.16305-16310.
[4] N. Magdelaine, L. Chaillous, and C. H. Moog, "A long-term model of the glucose-insulin dynamics of type 1 diabetes," IEEE Transactions on Biomedical Engineering, vol. 62, no. 6, pp. 1546-52, 2015.
[5] A. Zhakatayev, B. Rakhim, O. Adiyatov, A. Baimyshev, and H.A. Varol, "Successive linearization-based model predictive control of variable stiffness actuated robots". In 2017 IEEE international conference on advanced intelligent mechatronics (AIM), pp. 1774-1779, July 2017.
[6] M. E. Wilinska, L. J. Chassin, C. L. Acerini, J. M. Allen, D. B. Dunger, and R. Hovorka, "Simulation Environment to Evaluate Closed-Loop Insulin Delivery Systems in Type 1 Diabetes," 2010. [Online]. Available: www.journalofdst.org
[7] T. Homma and A. Saltelli, "Importance measures in global sensitivity analysis of nonlinear models," Reliab Eng Syst Saf, vol. 52, no. 1, pp. 1-17, Apr. 1996, doi: 10.1016/0951-8320(96)00002-6.
[8] J. Norton, "An introduction to sensitivity assessment of simulation models," Environmental Modelling & Software, vol. 69, pp. 166-174, Jul. 2015, doi: 10.1016/j.envsoft.2015.03.020.
[9] I. M. Sobol' and E. E. Myshetskaya, "Monte Carlo estimators for small sensitivity indices," Monte Carlo Methods Appl, vol. 13, no. 5-6, Jan. 2008, doi: 10.1515/mcma.2007.023
[10] Dalla-Man, C., Micheletto, F., Lv, D., Breton, M., Ko- vatchev, B., and Cobelli, C. (2014). The uva/padova type 1 diabetes simulator: new features. Journal of diabetes science and technology, 8(1), 26-34

## Claims

1. Computer- or processor-implemented method (100) for predicting a user's glycemic state within a predefined prediction time horizon, the method comprising the following steps:
a. Estimating the user's current metabolic state based on a current glucose monitoring user's data by means of an Extended Kalman Filter, wherein the hyperparameters of the Extended Kalman Filter are inferred from user's historical continuous glucose monitoring data and insulin and, advantageously meal records, including meal timing and composition (101);
b. Predicting the user's glycemic state within the predefined prediction time horizon based on the estimated current metabolic state and a personalized glucose prediction model, wherein the parameters of the glucose prediction model are determined for a specific demographic group except for n parameters, where n≥2, identified as being most influential for user-specific model personalization, and wherein the said n parameters are inferred from user's historical continuous glucose monitoring data and insulin and, advantageously meal records, including meal timing and composition (102).

2. Method (100) according to claim 1, wherein the most influential n parameters in the glucose prediction model are identified considering specific glucose-related states, such as postprandial and post-absorptive states, and events, such as used-induced correction boluses, using a time-dependent Sobol analysis.

3. Method (100) according to any one of the claims 1 or 2, wherein the hyperparameters of the Extended Kalman Filter and/or the parameters of the glucose prediction model are continuously adapted based on user's continuous glucose monitoring, meal and insulin records.

4. Method (100) according to any one of the preceding claims, wherein the glucose prediction model is the Hovorka glucose regulatory model, the Subcutaneous Oral Glucose Minimal Model (SOGMM), the Subcutaneous Oral Glucose Minimal Model (SOGMM) or the Madgelaine's Model.

5. Method (100) according to any one of the preceding claims, wherein the hyperparameters of the Extended Kalman Filter inferred from user's historical continuous glucose monitoring data and insulin and meal records including meal timing and composition are the process noise covariance matrix Q, the measurement noise covariance matrix R, and/or the initial state covariance matrix P0.

6. A method (200) for controlling an insulin delivery device using a control unit (300) comprising a Model Predictive Control (MPC) system, wherein the method comprises the following steps:
a. Acquiring a user's current glucose level, preferably via a continuous glucose monitor (201);
b. Predicting the user's glycemic state within a specified prediction time horizon by means of the method of any one of the claims 1 to 5 (202);
c. Determining optimal insulin dosage(s) within the specified prediction time horizon, to minimize glucose level deviations from a predefined target range (203);
d. Transmitting the determined optimal insulin dosage(s) to the insulin delivery device for administration to the user (204);
wherein steps a. to d. are repeated at a predetermined frequency (205).

7. Method (200) according to claim 6, wherein the glucose prediction model is a nonlinear model and wherein for each prediction of the user's glycemic state at step b., the model is linearized around the estimated current metabolic state.

8. Method (200) according to any one of the claims 6 or 7, wherein the prediction of the user's glycemic state at step b. incorporates user-provided meal announcements data.

9. Method (200) according to any one of the claims 6 to 8, wherein the optimal insulin dosage(s) determined at step c. is computed taking into account eventual user's manual insulin injection(s) and/or insulin injection(s) triggered by a hyperglycemia supervisor module (307) of the control unit (300).

10. Method (200) according to any one of the claims 6 to 9, comprising a step of evaluating the determined optimal insulin dosage(s) against one or more safety thresholds prior to transmission to the insulin delivery device for administration.

11. Method according to any one of the claims 6 to 10, further comprising:
i. Maintaining an estimate of Insulin On Board representing the residual effect of recently administered insulin
ii. At step c., incorporating the estimate of Insulin On Board, the current glucose level and the current glucose rate of change as constraint, to avoid excessive insulin stacking; and
iii. Updating the estimate of Insulin On Board after each administration at step d.

12. A control unit (300) communicatively coupled to an insulin delivery system and a continuous glucose monitoring device and comprising means adapted to execute the steps of the method of any one of the claims 6 to 11.

13. A computer program product comprising instructions to cause the system of claim 12 to execute the steps of the method of any one of the claims 6 to 11.

14. A computer-readable medium having stored thereon the computer program of claim 13.
